# EUROPEAN PATENT APPLICATION

(11) **EP 2 992 822 A1**
(43) Date of publication of application: **09.03.2016**
(21) Application number: 15466008.8
(22) Date of filing: 18.06.2015
(51) Int. Cl.: A61B 5/024, A61B 5/03, A61B 5/00, A61B 3/16, A61B 3/18

(54) **DEVICE FOR MEASURING AND MONITORING PRESSURE PULSES**

(30) Priority: 08.09.2014 CZ 20140610
(71) Applicant: Univerzita Karlova v Praze Lekarska Fakulta v Hradci Kralove, 500 38 Hradec Kralove (CZ)
(72) Inventor: Masín, Vladimír, 289 03 Mestec Králové (CZ); Rehák, Svatopluk, 544 64 Kocbere (CZ); Malec, Rudolf, 500 02 Hradec Králové (CZ); Vachek, Mikulás, 500 11 Hradec Králové (CZ); Bezrouk, Ales, 503 32 Hradec Králové (CZ); Záhora, Jirí, 500 04 Hradec Králové (CZ); Hanus, Josef, 500 08 Hradec Králové (CZ)

(57) **Abstract**

The device concerns the field of medical technology and consists of glasses (1) equipped with a sealed chamber (2) with valves (3) and a pressure sensor (4) connected to the input of the filtered power (9), which is connected to the signal filter (6) connected to the analogue/digital converter (7) and further to the computer (8). The filtered input power (9) can also be connected to the ECG (10) connected to the signal filter (6) connected to the analogue/digital converter (7) and further to the computer (8). The filtered input power (9) can be also connected to the sensor (12) of the perfusion connected to the power filter (5) connected to the analogue/digital convertor (7) and further to the computer (8).Valves (3) is usually connected to the actuating moduls (14) connected to the computer (8).

## Description

### Technical field

The invention related to the device concerns medical technology and provides non-invasive measuring and monitoring of pressure pulses and at the same time measures the intraocular pressure, intracranial pressure, quality of blood supply (perfusion) and collateral circulation.

### Existing technical background

Until now, devices measuring pulses of the cornea have utilised suction pads. Unfortunately, this method is painful for the patient. Intracranial pressure has always been measured in an invasive way, via a drilled probe. This method is also painful and requires anaesthesia. The quality of blood supply (perfusion) is measured by ultrasound or with the use of expensive magnetic resonance. The function of collateral circulation has always been examined by palpation only or by ultrasound, a technique requiring the usage of an expensive device and dependent on the subjective judgement of the practitioner. Moreover, high level ultrasound is required and this can lead to overheating or even micro-destruction of the tissue.

The goal of the invention is to create a non-invasive device which will provide accurate results of the measurement, and which will neither be expensive nor cause the patient pain.

### Background of the invention

The goals which have been mentioned have been achieved by constructing a device measuring and monitoring pressure pulses. The device consists of glasses equipped with a sealed chamber with valves and a pressure sensor. The sensor is connected to a filtered electric power supply. The glasses are also connected to a signal filter, which in turn is connected to an analogue/digital convertor leading to the computer. Filtered voltage is supplied to the pressure sensor in the sealed chamber and the signal from this pressure sensor is brought through the signal filter to the analogue/digital converter and from the converter to the computer for analysis of the measured data.

The filtered power supply can be connected to an ECG, which is in turn connected to the signal filter connected to the analogue/digital convertor and then to the computer. The filtered power is brought to the ECG from where the signal goes to the signal filter and from there to the analogue/digital converter and to the computer.

The filtered power supply can be also connected to the sensor of perfusion. From there the signal goes to the analogue/digital converter and to the computer.

The valwes is usually connected to the actuating moduls connected to the computer.

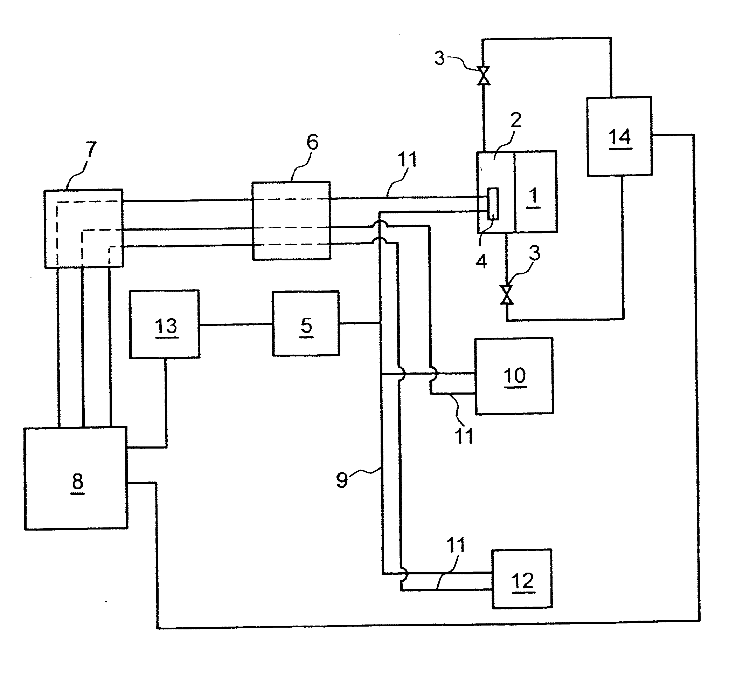

### The drawing

The connection of the device according to the invention is schematically shown in the enclosed drawing.

### Example of the invention

The device for measuring and monitoring pressure pulses consists of the power supply 13 connected to the power supply filter 5 from which cables lead the filtered voltage 9 to the sealed chamber 2 and supply the pressure sensor 4. This sealed chamber 2 is a part of the glasses 1 which the patient shall wear. This sealed chamber 2 is with valves 3 which is further connected to the actuating moduls 14 connected to the computer 8. The filtered voltage cables 9 go from the power supply filter 5 to the ECG 10 and to the blood perfusion sensor 12. The output cables 11 lead the outgoing signal from the pressure sensor 4, ECG 10 and the blood perfusion sensor 12 in the sealed chamber 2 of the glasses 1 to the signal filter 6, which is linked to the analogue/digital converter 7 and then to the computer 8.

The pressure signals are led via output cables 11 from the pressure sensor 4 in the sealed chamber 2 of the glasses 1, which the examined person is wearing on his head, to the signal filter 6, where they are filtered and conveyed to the analogue/digital converter 7 and then to the computer 8 where they are processed and analysed. The pressure signals coming out of the pressure sensor 4 can be amplified by the voltage brought from the power supply 13 and filtered in the input power filter 5 and linked to the pressure sensor 4 via the cables 9 conveying the filtered voltage. The pressure signals from the signal filter 6 are converted in the analogue/digital converter 7 and then go to the computer 8, where simultaneously are analysed signals brought from the ECG 10 and the blood perfusion sensor 12 via output cables 11 leading the signal through the filter of the signal 6 and the analogue/digital converter 7. The voltage brought to the ECG 10 and the sensor of perfusion 12 is also from the input power supply 13 and filtered in the input power filter 5. The device can be used for other types of examinations, for example intraocular pressure or measuring of cornea pulses, in which case only the glasses 1 are put on the head of the examined person. In the case of collateral circulation, perfusion or intracranial pressure the readings from the glasses 1, ECG 10 and the perfusion sensor 12 are processed and analysed in the computer. Through this computerised analysis we achieve non-invasive and precise results. This way of examination has another advantage: it is possible to use it for retrograde check-ups during head surgery and for continuous check-ups for patients in a lying position.

## Claims

1. A device measuring and monitoring the pressure pulses **characterized in that** it comprises of glasses (1) equipped with a sealed chamber (2) with valves (3) and a pressure sensor (4) connected to the input of the filtered power (9) and connected to the signal filter (6), which is linked to the analogue/digital converter (7) leading to the computer (8).

2. The device as described in the claim 1 **characterized in that** the input of filtered power (9) is also connected to the ECG (10), which is connected to the signal filter (6) which is further connected to the analogue/digital converter (7) and to the computer (8).

3. The device as described in the claims 1 or 2 **characrterized** in that the input of filtered power (9) is also connected to the perfusion sensor (12), which is connected to the signal filter (6) which is further connected to the analogue/digital converter (7) and to the computer (8).

4. The device according to any of the preceding claims **characterized in that** valves ( 3 ) is connected to the actuating moduls ( 14 ) connected to the computer (8).
